# EUROPÄISCHE PATENTSCHRIFT

(11) **EP 1 178 783 B1**
(45) Veröffentlichungstag und Bekanntmachung des Hinweises auf die Patenterteilung: **21.04.2004**
(21) Anmeldenummer: 00931238.0
(22) Anmeldetag: 17.05.2000
(51) Int. Cl.: A61K 9/70

(54) **VORRICHTUNG UND VERWENDUNG ZUR STEIGERUNG DER TRANSDERMALEN PERMEATION VON ARZNEISTOFFEN**
DEVICE AND USE FOR INCREASING THE TRANSDERMAL PERMEATION OF MEDICAMENTS
DISPOSITIF ET UTILISATION POUR AUGMENTER LA PERMEATION TRANSDERMIQUE DE MEDICAMENTS

(30) Priorität: 21.05.1999 DE 19923427
(43) Veröffentlichungstag der Anmeldung: 13.02.2002
(73) Patentinhaber: LTS Lohmann Therapie-Systeme AG, 56626 Andernach (DE)
(72) Erfinder: KOCH, Andreas, D-56581 Melsbach (DE); VON FALKENHAUSEN, Christian, D-53340 Meckenheim (DE); MATUSCH, Rudolf, D-35041 Marburg (DE); ADAM, Bernd, D-34613 Treysa (DE)
(74) Vertreter: Schmidt, Werner, Dr.
(86) Internationale Anmeldenummer: PCT/EP2000/004460
(87) Internationale Veröffentlichungsnummer: WO 2000/071100

(56) Entgegenhaltungen:
- EP-A- 0 464 573
- WO-A-97/13482
- DE-A- 19 738 855

## Beschreibung

Die Erfindung betrifft eine Vorrichtung und ein Verfahren zur Steigerung der dermalen und / oder transdermalen Permeation von Arzneistoffen.

Die transdermale Verabreichung von pharmazeutischen Wirkstoffen ist seit der ersten kommerziellen Nutzung eines scopolaminhaltigen Transdermalen Therapeutischen Systems (Scopoderm TTS) bekannt. Auch andere Wirkstoffe (Nitroglycerin, Estradiol, Clonidin, Isosorbiddinitrat, Fentanyl, Nicotin, Norethisteron etc.) werden mittlerweile in Form eines solchen TTS angeboten. Diese wirkstoffhaltigen Pflaster werden auf die Haut eines Patienten aufgeklebt. Der Wirkstoff wird dann in kontrollierter Weise aus dem TTS an die Haut des Patienten abgegeben, wandert durch die verschiedenen Schichten der Haut und tritt schließlich in den Blutkreislauf ein.

Ein Problem bei dieser Art der Verabreichung eines systemisch wirksamen Stoffes liegt sehr häufig darin, daß der durch die unterschiedlich aufgebauten Schichten der Haut führende Weg des Wirkstoffs recht lang und der Transport der Wirkstoffs bis zum Eintritt in den Blutkreislauf zeitraubend ist. Dies hat mehrere Ursachen. Zum einen besitzt die Haut eine natürliche Barrierefunktion gegenüber dem Eindringen von Fremdsubstanzen in den Körper. Andererseits besitzen Substanzen mit sehr großem Molekülradius nur einen geringen Diffusionskoeffizient. Dieser stellt ein Maß für die Fähigkeit eines Stoffes dar, innerhalb eines Mediums zu wandern, im speziellen also das Permeationsverhalten innerhalb der Hautschichten. Und schließlich können physikalisch-chemische Wechselwirkungen (zum Beispiel: polare Wechselwirkungen, Dipol-Dipol-Wechselwirkungen etc.) zwischen dem Wirkstoff und den verschiedenen Bestandteilen der unterschiedlichen Hautschichten die Diffusionsgeschwindigkeit eines Wirkstoffes in der Haut herabsetzen.

Alle diese Phänomene können dazu führen, daß die perkutane Absorption und die anschließende Resorption des Wirkstoffs derart langsam erfolgt, daß das Eintreten einer pharmakologischen Wirkung erst mit deutlicher Verzögerung erfolgt. Diesen Zeitraum zwischen Applikation eines TTS auf der Haut eines Patienten und dem Eintreten der pharmakologischen Wirkung bezeichnet der Fachmann als "lag-time".

Die Verbesserung der Permeation eines transdermal applizierbaren Wirkstoffs durch die Haut (engl. "percutaneous absorption enhancement") ist daher fast logischerweise Gegenstand umfangreicher Forschungsarbeiten, insbesondere der pharmazeutischen Industrie gewesen. Dies hat zur Anwendung verschiedenartigster, die Hautdurchdringung verbessernder Stoffe (sog. Enhancer) und zur Entwicklung spezieller Substanzen, z. B. Azone®, geführt. Zu Enhancern zählen Alkohole, Amide, Aminosäuren, Derivate von Azone®, etherische Öle, Fettsäuren und Fettsäurederivate wie Fettsäureester, Makrocyclen, Phospholipide, Pyrrolidone, Sulfoxide und andere.

Die Erhöhung der Hautdurchdringung (genauer: die Beschleunigung des Eintritts in und des Durchwandems eines Wirkstoffs durch die verschiedenen Hautschichten und somit schließlich auch die Erhöhung der transdermalen Absorptionsrate) wird bei der Gabe solcher Enhancer im wesentlichen dadurch erreicht, daß diese Enhancer in die obersten Schichten der Haut eindringen und hier die Struktur der Haut so verändern, daß ihre Barrierefunktion nicht mehr in vollem Umfang wirksam ist. Man erklärt sich diese Wirkungsweise auf molekularer Ebene damit, daß sich die Enhancer in biologische Membranen einlagern und die natürliche Struktur von Lipiden und Proteinen verändern, z. B. zum Anschwellen bringen. Der auf die Haut aufgebrachte Wirkstoff kann nun leichter, d. h. schneller durch die verschiedenen Schichten der Haut wandern.

WO 97/13482 beschreibt ein transdermales Transportsystem enthaltend Vasodilator Wirkstoff, Enhancer und wasserläsliches Gummi.

Die Anwendung der im Stand der Technik bekannten Enhancer besitzt jedoch auch zahlreiche Nachteile. Zunächst darf der Enhancer nicht toxisch sein und darf keine physiologischen Nebenwirkungen verursachen. Außerdem dürfen keine chemischen oder physikalischen Inkompatibilitäten zwischen Enhancer und dem Wirkstoff bzw. den weiteren, das TTS aufbauenden Materialien bestehen. Schließlich muß der Enhancer in einer solchen Menge im TTS enthalten sein, daß über dessen gesamte Anwendungsdauer (im allgemeinen: 16 bis 24 Stunden, es gibt jedoch auch schon 3-Tage-Pflaster) der permeationsverstärkende Effekt erhalten bleibt. Dies führt jedoch oft zwangsläufig dazu, daß dieser Enhancer ebenfalls in solchen Mengen an den Patienten verabreicht wird, daß ein systemischer Effekt verursacht wird.

Aufgabe der vorliegende Erfindung ist es, das Eindringen eines Wirkstoffs in die Haut und / oder das Durchwandern dieses Wirkstoffs durch die verschiedenen Schichten der Haut und / oder den Abtransport dieses Wirkstoffs durch das Blutkapillarsystem der Dermis und / oder die Blutgefäße der Hypodermis derart zu beschleunigen, daß eine schnellere Absorption und Resorption durch die Haut erfolgt und somit eine Verkürzung der "lag-time" erzielt wird. Insbesondere sollen Wirkstoffe, die von Natur aus bei Absorption und Resorption durch die Haut eine lange "lag-time" zeigen, der praktischen transdermalen Anwendung zugänglich gemacht werden.

Gelöst wird die Aufgabe durch eine Vorrichtung, die dadurch gekennzeichnet ist, daß sie eine Komponente enthält, die eine lokale Temperaturerhöhung der Haut bewirkt und / oder die Durchblutung steigert. Die erfindungsgemäße Vorrichtung ist ein Pflaster wie in den Ansprüchen definiert.

Die Verwendung von Stoffen, die eine lokale Temperaturerhöhung der Haut bewirken zum Zwecke der Erhöhung der Geschwindigkeit des Eindringen eines Wirkstoffs in die Haut und / oder des Durchwanderns dieses Wirkstoffs durch die verschiedenen Schichten der Haut und / oder des Abtransports dieses Wirkstoffs durch das blutkapillarsystem der Dermis und / oder die Blutgefäße der Hypodermis ist ebenfalls Gegenstand der vorliegenden Erfindung. Es kann sowohl ein Kurzzeiteffekt (das heißt bis zum Eintreten der pharmakologischen Wirkung) als auch ein Langzeiteffekt (das heißt während der gesamten Dauer der transdermalen Applikation des betreffenden, transdermal zu applizierenden Wirkstoffs) erzielt werden.

Der weiteren Erläuterung der Erfindung dienen die folgenden Definitionen:

Unter dem Begriff der Haut wird die unverletzte, d. h. nicht-geschädigte und normal behaarte, unrasierte Haut eines Säugetieres, insbesondere die eines Menschen verstanden. Schleimhaut zählt nicht zur Haut im Sinne dieser Erfindung. Die normale Haut besteht im wesentlichen aus drei Schichten: der (außen liegenden) Epidermis, der Dermis und der darunterliegenden Hypodermis. Die Hypodermis wird man auch Unterhaut oder subcutis genannt. Die Epidermis wiederum setzt sich aus stratum comeum, stratum lucidum, stratum granulosum, stratum spinosum und stratum germativum zusammen. Die Dermis enthält unter anderem das Blutkapillarsystem. In der Hypodermis befinden sich die Blutgefäße.

Als Penetration bezeichnet man das Eindringen eines Wirkstoffs in die äußeren Schichten der Haut, insbesondere in die der Epidermis. Unter Permeation versteht man das Hindurchwandern des Wirkstoffs durch die Haut, insbesondere durch die Epidermis (einschließlich des stratum corneums) und die Dermis.

Unter einer dermalen Applikation im Sinne der vorliegenden Beschreibung ist zu verstehen, daß ein Wirkstoff auf die Haut aufgebracht wird und in der Epidermis und / oder der Dermis eine lokale oder regionale (also topische) Wirkung entfaltet. Unter einer transdermalen Applikation im Sinne der vorliegenden Beschreibung ist zu verstehen, daß ein Wirkstoff auf die Haut aufgebracht wird, durch die Epidermis und / oder die Dermis in die Blutbahn oder in das Lymphsystem gelangt, sich von dort aus über den Gesamtorganismus verteilt und am Zielort eine systemische Wirkung entfaltet. Mit transdermaler Absorption oder Resorption bezeichnet man die Aufnahme eines Wirkstoffs durch die unter der Haut liegenden Blutgefäße nach transdermaler Applikation, wonach eine systemische Wirkung des aufgenommenen Wirkstoffs ermöglicht wird.

Wirkstoffe im Sinne der vorliegenden Erfindung sind im wesentlichen pharmazeutische und / oder kosmetische Wirkstoffe, die zur Vorbeugung, Linderung, Heilung oder Erkennung von Erkrankungen dienen. Hierunter sind auch solche Stoffe zu verstehen, die eine topische und / oder systemische Wirkung erzielen können. Wirkstoffe sind dem Fachmann bekannt und können Standardwerken entnommen werden, wie z. B. der "Roten Liste" (pharmazeutische Wirkstoffe) und der "Grünen Liste" (kosmetische Wirkstoffe). Weitere Wirkstoffe sind in US 4,557,934 genannt und in den dort (Sp. 10, Z. 18-35) zitierten Quellen. Grundsätzlich ist dabei die Art des Wirkstoffs für die vorliegende Erfindung nicht wesentlich.

Zu den Wirkstoffen, die von Natur aus bei Absorption und Resorption durch die Haut eine lange "lag-time" zeigen, zählen: Opiate wie z. B. Morphin, Diamorphin, Buprenorphin; Fentanyl, Oestrogene wie z. B. 17β-Estradiol; Hormone wie z. B. Testosteron, Norethisteron, Gestagene; Peptide wie z. B. Insulin; Analgetika; Alkaloide. Diese Art von systemischen Wirkstoffen kann durch den Gegenstand der Erfindung in besonders vorteilhafter Weise der transdermalen Anwendung zugänglich gemacht werden.

Mit der Komponente, die eine lokale Temperaturerhöhung und / oder eine Durchblutungssteigerung der Haut bewirkt ist eine Substanz oder eine Mischung von Substanzen gemeint, die beim Aufbringen auf die Haut eine lokale Erwärmung der Haut zur Folge hat. Zu solchen Substanzen zählen insbesondere Rubefacientia, Vasodilatatoren und hyperämisierende Stoffe.

Unter Rubefacientia versteht man Stoffe, die infolge Hyperämie eine hautreizende und / oder hautrötende Wirkung erzielen. Rubefacientia sind somit eine besondere Gruppe von hyperämisierenden Stoffen. Hierzu zählen z. B. Pelargonsäurevanillylamid, Cayennepfefferölharz, Extract. Fruct. Capsici, Tinct, Capsici, Cayennepfefferextrakt, Cayennepfeffer und Capsaicin.

Unter Vasodilatatoren versteht man gefäßerweiterende Stoffe, die z. B. durch Erschlaffung der Gefäßmuskulatur eine Erweiterung der Blutgefäße bewirken. Hierbei tritt auch oft eine Blutdrucksenkung auf. Dies erfolgt natürlich auch bei den peripheren Blutgefäßen, wodurch eine Mehrdurchblutung des betroffenen Gebietes bewirkt wird. Zu den Vasodilatatoren zählen: Nicotinsäure; Derivate der Nicotinsäure wie z. B. Nicotinylalkohol, Nicotinsäurebenzylester, Nicotinamid etc.; Adenosin-Verbindungen; Xanthin, Derivate des Xanthins wie Coffein, etc., Arnikaextrakt, Arnikablütenextrakt, Pyridyl-3-carbinol und seine Salze, Chamomilla, Phytolacca, Guaiacum, Cinnabaris, Kreosotum, Luffa operculata, Tinct. rhus. toxicodendron.

Unter hyperämisierende Stoffen versteht man durchblutungssteigernde Substanzen. Die verstärkte Durchblutung wird dadurch hervorgerufen, daß ein vermehrter Blutzufluß (reaktive Hyperämie) oder ein verringerter Blutabfluß (Stauungs-Hyperämie) stattfindet. Dies kann unterschiedliche Ursachen haben. Zu den hyperämisierenden Stoffen zählen: etherische Öle, Menthol, Campher, Nicotinsäureester, Salicylsäureester, Hydroxyethylsalicylat, Methylsalicylat, ortho-Carbamoylphenoxyessigsäure.

Der Wärmeeffekt in der Haut wird also im wesentlichen dadurch erzielt, daß die Durchblutung der peripheren Blutgefäße (Kapillarsystem) verbessert wird. Er beruht nicht darauf, daß von außen eine Wärmewirkung in Form von z. B. Wärmeleitung, Ultraschall, Wärmestrahlung etc. auf die Haut ausgeübt wird. Es findet kein Energietransport aus einer äußeren Energiequelle in die Haut statt.

Das Pflaster, d. h. ein dermales oder transdermales therapeutisches System enthält folgende Konstruktionselemente: eine wirkstoffundurchlässige Rückschicht, ein wirkstoffhaltiges Reservoir, eine die Freisetzung des Wirkstoffs kontrollierende (semipermeable oder mikroporöse) Membran, eine wirkstoffhaltige Kleberschicht und eine wiederablösbare Schutzschicht (sog. "release liner").

Zur vermeidung von Inkompatibilitäten, ist die Komponente, die eine lokale Temperaturerhöhung der Haut bewirkt, in einem separaten, gegenüber den wirkstoffhaltigen Konstruktionselementen räumlichen getrennten Konstruktionselement der Vorrichtung enthalten.

Bevorzugt ist eine Ausführungsform, bei der um ein inneres Segment, welches den transdermal zu applizierenden Wirkstoff enthält, ein äußeres Segment, welches die Komponente, die eine lokale Temperaturerhöhung und / oder Durchblutungssteigerung der Haut bewirkt enthält, herum angeordnet ist. Die Form des inneren Segments ist im Prinzip egal, sie kann kreisförmig, rechteckig oder quadratisch sein. Die Form des äußeren Segment ist im Prinzip ebenfalls egal, sie kann auch kreisförmig, rechteckig oder quadratisch sein. Das äußere Segment kann aber auch ringförmig oder in zwei oder mehreren, zum Beispiel rechteckigen oder halbkreisförmigen Abschnitten um das innere Segment herum angeordnet sein. Diese Ausführungsform ist deshalb besonders geeignet, weil sie von der Ausbildung eines horizontalen Konzentrationsgradienten in der Epidermis und der Dermis/Subcutis profitiert. Diese sogenannte Lateraldiffusion ist zwar aus dem Aufbau des stratum comeums mit seinem lamellenartigen Lipid-Wasser-Doppelschichten ableitbar, für eine praktische Umsetzung hinsichtlich der Erhöhung der Geschwindigkeit des Eindringens in die Haut und / oder des Durchwanderns durch die Haut, aber insbesondere zur Erhöhung der transdermalen Absorptionsrate eines auf die Haut applizierten Wirkstoffs ist dieses Phänomen überraschenderweise bisher jedoch noch nicht von der Fachwelt in Betracht gezogen worden.

In dieser vorteilhaften Ausführungsform überragt das äußere Segment das innere Segment deutlich, zum Beipiel um einige Millimeter (z. B.: 5 bis 25) oder um die Länge des Durchmessers des inneren Segments. Auf diese Weise wirkt das Prinzip, das für die Steigerung der transdermalen Absorptionsrate des Wirkstoffs verantwortlich ist, auf eine über die reine Abgabefläche des transdermal applizierten Wirkstoffs hinausragende Fläche.

Die Fläche der Haftkleberschicht bzw. des äußeren Segments, welche die Komponente, die eine lokale Temperaturerhöhung und / oder Erhöhung der Durchblutung der Haut bewirkt, enthält, ist im allgemeinen kleiner als 100cm².

Die Herstellung einer solchen bevorzugten Ausführungsform kann ebenfalls nach dem Fachmann bekannten Verfahren erfolgen, wie z. B. in DE 37 14 140, DE 38 09 978 und DE 41 10 027 beschrieben ist. Es ist klar, daß bei der Herstellung des äußeren Segments die Komponente, die eine lokale Temperaturerhöhung der Haut bewirkt, in dem entsprechenden Arbeitsschritt in die das äußere Segment bildende Matrix eingearbeitet werden muß. Die räumliche Trennung der Segmente mit der Komponente, die eine lokale Temperaturerhöhung und / oder Durchblutungsteigerung der Haut bewirkt, und der Segmente mit dem transdermal zu applizierenden Wirkstoff wird durch horizontale oder vertikale Sperrschichten oder Materiallücken erzielt.

Die Wirkung der erfindungsgemäßen Vorrichtung kann sich - je nach Gehalt der Komponente, die die eine lokale Temperaturerhöhung und / oder Durchblutungsteigerung der Haut bewirkt, - über einen längeren Zeitraum erstrecken, z. B. etwa 8, 16, 24, 48 oder sogar 72 Stunden. Die erfindungsgemäße Vorrichtung wird bei normalen Bedingungen auf die Haut des Patienten aufgetragen, also bei normaler, d. h. nicht erhöhter Hautaußentemperatur. Die erfindungsgemäße Vorrichtung kann auch vor und während einer Iontophorese-Behandlung angewendet werden.

Der Gehalt des Stoffes, der eine lokale Temperaturerhöhung und / oder Durchblutungssteigerung der Haut bewirken kann innerhalb der Vorrichtung richtet sich danach, ob ein Kurzzeiteffekt bis zum Eintreten der pharmakologischen Wirkung oder ein Langzeiteffekt während der gesamten Dauer der transdermalen Applikation des betreffenden, transdermal zu applizierenden Wirkstoffs erwünscht ist. Ausserdem richtet er sich im konkreten Fall nach den speziellen Stoffeigenschaften. Im allgemein liegt der Gehalt an diesem Stoff innerhalb der Vorrichtung daher zwischen 0,5 und 20 Gew.-%.

Die erfindungsgemäße Vorrichtung ist vorzugsweise frei von Permeationsenhancem, d. h. solchen Stoffen, die durch Eingriff in die Struktur der Haut eine Verbesserung der Hautpermeation eines dermal applizierten Wirkstoffs erzielen, kann aber ggf. solche Stoffe enthalten. Eine Liste dieser Stoffe ist dem Artikel von D. W. Osborne und J. J. Hill über "Skin Penetration Enhancers cited in the Technical Literature" der internet-Website http://pharmtech.com/technical/osborne/osborne.htm zu entnehmen.

Die folgenden Beispiele dienen der Erläuterung der erfindungsgemäßen Wirkungsweise.

### Beispiel 1:

Drei Muster eines kommerziell erhältlichen estradiolhaltigen Wirkstoffpflasters (Vivelle®) wurden jeweils auf 4,52 cm² große kreisförmige Proben einer Humanvollhaut mittig appliziert. Die so präparierten Hautproben wurden 72 h lang in modifizierte FRANZ-Zellen gegeben, wobei sich zur Vermeidung von Artefakten durch Rückdiffusion in den Zellen kein Akzeptormedium befand. Nach Ende dieser Zeit wurden die Wirkstoffpflaster entfernt und die Haut unter der Applikationsstelle exakt ausgestanzt. Es wurden weitere Kreissegmente aus der Hautprobe ausgestanzt, die jeweils immer weiter weg vom Kernsegment, das heißt der eigentlichen Abgabefläche des TTS, lagen.

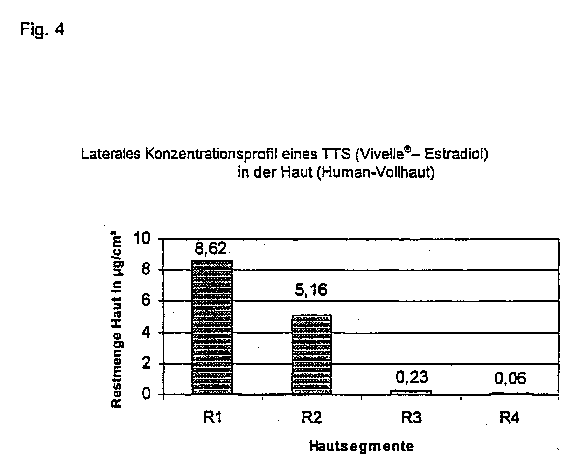

Die so erhaltenen Hautproben wurden anschließend 16 h lang mit Methanol extrahiert und einer Restmengenbestimmung bezüglich Oestradiol unterzogen. Hierbei konnte ein horizontales Konzentrationsprofil ermittelt werden, welches auch statistisch signifikant war. Die Ergebnisse der Restmengenbestimmung sind in Tabelle 1 enthalten. Das ermittelte horizontale Konzentrationsprofil wird in Abbildung Fig. 4 wiedergegeben.

**Tabelle 1:**

| Hautsegment | R1 | R2 | R3 | R4 |
|---|---|---|---|---|
| Restgehalt in µg/cm² | 8,62 | 5,16 | 0,23 | 0,06 |
| zur Erläuterung: | | | | |
| R 1 = Hautsegment direkt unter der TTS - Applikationsfläche (= 1,13 cm²) | | | | |
| R 2 = 1. Hautsegment außerhalb des TTS-Kernsegments (= 1,41 cm²) | | | | |
| R 3 = 2. Hautsegment außerhalb des TTS-Kernsegments (= 1,98 cm²) | | | | |
| R 4 = 3. Hautsegment außerhalb des TTS-Kernsegments (= 2,54 cm²) | | | | |

### Beispiel 2:

An zwei Gruppen von Ratten mit je n = 6 Tieren wurde ein in vivo-Tierversuch vorgenommen. Den Tieren beider Gruppen wurden je ein TTS mit einem schlecht resorbierbaren Wirkstoff (Morphinbase) aufgeklebt. Den Tieren der ersten Versuchsgruppe wurde über das eigentliche wirkstoffabgebende TTS ein Überpflaster mit einer wirksamen Menge einer Komponente, die eine lokale Temperaturerhöhung der Haut bewirkt, appliziert (ABC-Pflaster der Firma Beiersdorf, Hamburg). Die wirksamen Stoffe dieses Überpflasters sind Capsaicin und Arnika-Extrakt. Der flächenmäßige Hautkontakt des Überpflasters im Vergleich zu dem des wirkstoffabgebenden TTS betrug 3 zu 1.

Die Tiere der zweiten Versuchsgruppe (sog. Kontrollgruppe) erhielten als Überpflaster eine selbstklebende Okklusivfolie ohne eine Komponente, die eine lokale Temperaturerhöhung der Haut bewirkt (Opraflex der Fa. Lohmann, Neuwied). Der flächenmäßige Hautkontakt des Überpflasters im Vergleich zu dem des wirkstoffabgebenden TTS betrug ebenfalls 3 zu 1.

Nach Ende der 24-stündigen Tragedauer wurden die Pflasterverbände abgenommen und einer Restgehaltsbestimmung auf Morphinbase unterzogen. Die Ergebnisse sind in der Tabelle 2 zusammengestellt und zeigen, daß durch die Verwendung des Überpflasters mit einer wirksamen Menge einer Komponente, die eine lokale Temperaturerhöhung der Haut bewirkt, die transdermale Absorptionsrate von 5,7 auf 26,4% erhöht werden konnte.

**Tabelle 2:**

| Versuchsgruppe mit "Opraflex" - Überpflaster | | | |
|---|---|---|---|
| TTS | Ausgangsgehalt | Restgehalt | freigesetzt |
| Verum 1 | 546,7 µg/cm² | 506,6 µg/cm² | 40,1 µg/cm² |
| Verum 2 | 546,7 µg/cm² | 514,2 µg/cm² | 32,5 µg/cm² |
| Verum 3 | 546,7 µg/cm² | 538,8 µg/cm² | 7,9 µg/cm² |
| Verum 4 | 546,7 µg/cm² | 528,0 µg/cm² | 18,7 µg/cm² |
| Verum 5 | 546,7 µg/cm² | 494,9 µg/cm² | 51,8 µg/cm² |
| Verum 6 | 546,7 µg/cm² | 510,0 µg/cm² | 36,7 µg/cm² |
| Mittelwert: | | 515,4 µg/cm² | 31,3 µg/cm² |

Daraus resultiert eine relative Absorptionsrate von 5,72 %.

Versuchsgruppe mit "ABC-Wärme-Pflaster N" - Überpflaster

| TTS | Ausgangsgehalt | Restgehalt | freigesetzt |
|---|---|---|---|
| Verum 1 | 546,7 µg/cm² | 364,7 µg/cm² | 182,0 µg/cm² |
| Verum 2 | 546,7 µg/cm² | 354,6 µg/cm² | 192,1 µg/cm² |
| Verum 3 | 546,7 µg/cm² | 442,9 µg/cm² | 103,8 µg/cm² |
| Verum 4 | 546,7 µg/cm² | 447,9 µg/cm² | 98,8 µg/cm² |
| Verum 5 | 546,7 µg/cm² | 446,8 µg/cm² | 99,9 µg/cm² |
| Verum 6 | 546,7 µg/cm² | 359,0 µg/cm² | 187,7 µg/cm² |
| Mittelwert: | | 402,7 µg/cm² | 144,1 µg/cm² |

Daraus resultiert eine relative Absorptionsrate von 26,4 %.

Dieses Ergebnis ist umso erstaunlicher, da es sich bei diesem Verfahren zur Verbesserung der Permeation eines transdermal applizierbaren Wirkstoffs durch die Haut weder um ein chemisches noch um ein anderes invasives Verfahren handelt, das die Haut nachhaltig schädigt. Es erfolgt also kein Eingriff in die Struktur der Haut, insbesondere der Epidermis, so daß auch nicht der "Selbstreparaturmechanismus" der Haut aktiviert werden muß.

Die Vorrichtung und das Verfahren bewirken also aufgrund einer lokalen Temperaturerhöhung und / oder Durchblutungsteigerung der Haut eine Erhöhung der Geschwindigkeit des Eindringens in die Haut und / oder eine Erhöhung des Durchwandems durch die Haut und / oder eine Erhöhung der transdermalen Absorptionsrate eines auf die Haut applizierten Wirkstoffs.

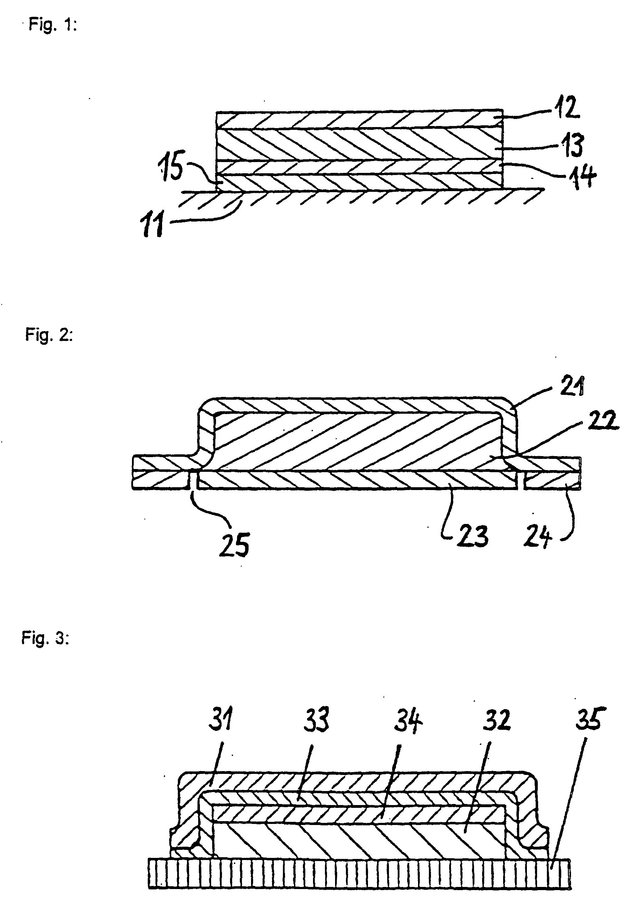

Anhand der folgenden Figuren 1 bis 3 werden erfindungsgemäße Ausführungsformen erläutert.

Fig. 1 (nicht erfindungegemäß) zeigt eine Ausführungsform, bei der die Komponente, die eine lokale Temperaturerhöhung und / oder Durchblutungsteigerung der Haut bewirkt, im demselben Reservoir und derselben Haftkleberschicht enthalten ist wie der transdermal zu applizierende Wirkstoff. Die Bezugszeichen haben folgende Bedeutung: 11 = Haut, 12 = wirkstoffundurchlässige Rückschicht, 13 = Reservoir, enthaltend den transdermal zu applizierenden Wirkstoff und gegebenenfalls die Komponente, die eine lokale Temperaturerhöhung und / oder Durchblutungssteigerung der Haut bewirkt, 14 = semipermeable oder mikroporöse Membran zur Steuerung der Wirkstofffreisetzung, 15 = Haftkleberschicht, enthaltend den transdermal zu applizierenden Wirkstoff und die Komponente, die eine lokale Temperaturerhöhung und / oder Durchblutungssteigerung der Haut bewirkt.

Fig. 2 zeigt eine Ausführungsform, bei der die Komponente, die eine lokale Temperaturerhöhung und / oder Durchblutungsteigerung der Haut bewirkt, durch eine vertikale Sperrschicht räumlich getrennt ist von dem Reservoir und der Haftkleberschicht, in denen der transdermal zu applizierende Wirkstoff enthalten ist. Die Bezugszeichen haben folgende Bedeutung: 21 = wirkstoffundurchlässige Rückschicht, 22 = Reservoir, enthaltend den transdermal zu applizierenden Wirkstoff, jedoch frei von der Komponente, die eine lokale Temperaturerhöhung und / oder Durchblutungssteigerung der Haut bewirkt, 23 = Haftkleberschicht, enthaltend den transdermal zu applizierenden Wirkstoff, jedoch frei von der Komponente, die eine lokale Temperaturerhöhung und / oder Durchblutungssteigerung der Haut bewirkt, 24 = Haftkleberschicht, enthaltend die Komponente, die eine lokale Temperaturerhöhung und / oder Durchblutungssteigerung der Haut bewirkt, 25 = vertikale Sperrschicht.

Fig. 3 zeigt eine Ausführungsform, bei der die Komponente, die eine lokale Temperaturerhöhung und / oder Durchblutungsteigerung der Haut bewirkt, durch eine horizontale Sperrschicht räumlich getrennt ist von dem Reservoir bzw. der Haftkleberschicht, in der der transdermal zu applizierende Wirkstoff enthalten ist. Die Bezugszeichen haben folgende Bedeutung: 31 = wirkstoffundurchlässige Rückschicht, 32 = Reservoir bzw. Haftkleberschicht, enthaltend den transdermal zu applizierenden Wirkstoff, jedoch frei von der Komponente, die eine lokale Temperaturerhöhung und / oder Durchblutungssteigerung der Haut bewirkt, 33 = Haftkleberschicht, enthaltend die Komponente, die eine lokale Temperaturerhöhung und / oder Durchblutungssteigerung der Haut bewirkt, 34 = horizontale Sperrschicht, 35 = Haut.

## Patentansprüche

1. Vorrichtung in Form eines Pflasters zur Erhöhung der Geschwindigkeit des Eindringens in die Haut und / oder des Durchwanderns durch die Haut und / oder der transdermalen Absorptionsrate von einem auf die Haut applizierten Wirkstoff, wobei das Pflaster schichtförmig aufgebaut ist, mindestens eine Schicht umfasst, die eine Komponente enthält, die eine lokale Temperaturerhöhung und / oder Durchblutungssteigerung der Haut bewirkt und mindestens eine Schicht umfasst, die mindestens einen pharmazeutischen und / oder kosmetischen Wirkstoff enthält, der eine systemische Wirkung besitzt, **dadurch gekennzeichnet, dass** die besagten Schichten räumlich voneinander getrennt sind.

2. Vorrichtung nach Anspruch 1, **dadurch gekennzeichnet, dass** sie eine Rückschicht enthält, die für die eine lokale Temperaturerhöhung bewirkende Komponente undurchlässig ist oder ein Austreten dieser Komponente nach der hautabgewandten Seite verhindert.

3. Vorrichtung nach Anspruch 1 oder 2, **dadurch gekennzeichnet, dass** sie eine haftklebende Schicht enthält, die sich vollflächig über die Unterseite der Vorrichtung erstreckt oder sich nur am Rand der Unterseite der Vorrichtung befindet.

4. Vorrichtung nach einem der vorangehenden Ansprüche, **dadurch gekennzeichnet, dass** die räumliche Trennung durch eine horizontale Sperrschicht, eine vertikale Sperrschicht oder eine Materiallücke erreicht wird.

5. Vorrichtung nach einem der vorangehenden Ansprüche, **dadurch gekennzeichnet, dass** die Komponente, die eine lokale Temperaturerhöhung und / oder Durchblutungssteigerung der Haut bewirkt, aus der Gruppe der Rubefacientia und / oder der Vasodilatatoren und / oder der hyperämisierenden Stoffe ausgewählt ist.

6. Vorrichtung nach einem der vorangehenden Ansprüche, **dadurch gekennzeichnet, dass** die Komponente, die eine lokale Temperaturerhöhung und / oder Durchblutungssteigerung der Haut bewirkt, Pelargonsäurevanillylamid, Cayennepfefferölharz, Extract. Fruct. Capsici, Tinct, Capsici, Cayennepfefferextrakt, Cayennepfeffer, Capsaicin oder eine Kombination davon enthält.

7. Vorrichtung nach einem der vorangehenden Ansprüche, **dadurch gekennzeichnet, dass** die Komponente, die eine lokale Temperaturerhöhung und / oder Durchblutungssteigerung der Haut bewirkt, Nicotinsäure; Derivate der Nicotinsäure, Nicotinylalkohol, Nicotinsäurebenzylester, Nicotinamid, Adenosin-Verbindungen; Xanthin, Derivate des Xanthins, Coffein, Arnikaextrakt, Arnikablütenextrakt, Pyridyl-3-carbinol und seine Salze, Chamomilla, Phytolacca, Guaiacum, Cinnabaris, Kreosotum, Luffa operculata, Tinct. rhus. toxicodendron oder Kombinationen davon enthält.

8. Vorrichtung nach einem der vorangehenden Ansprüche, **dadurch gekennzeichnet, dass** die Komponente, die eine lokale Temperaturerhöhung und / oder Durchblutungssteigerung der Haut bewirkt, etherische Öle, Menthol, Campher, Nicotinsäureester, Salicylsäureester, Hydroxyethylsalicylat, Methylsalicylat, ortho-Carbamoylphenoxyessigsäure oder Kombinationen davon enthält.

9. Vorrichtung nach einem der vorangehenden Ansprüche, **dadurch gekennzeichnet, dass** der auf die Haut applizierte systemische Wirkstoff ein pharmazeutischer Wirkstoff aus der Gruppe umfassend Opiate, Morphin, Diamorphin, Buprenorphin, Fentanyl, Oestrogene, 17β-Estradiol, Hormone, Testosteron, Norethisteron, Gestagene, Peptide, Insulin, Analgetika und Alkaloide ist.

10. Vorrichtung nach einem der vorangehenden Ansprüche, **dadurch gekennzeichnet, dass** um ein inneres Segment, welches den mindestens einen pharmazeutischen und / oder kosmetischen Wirkstoff enthält ein äußeres Segment angeordnet ist, welches die Komponente enthält, die eine lokale Temperaturerhöhung und / oder Durchblutungssteigerung der Haut bewirkt.

11. Verwendung einer Komponente, die zu einer lokalen Temperaturerhöhung und / oder Durchbiutungssteigerung der Haut befähigt ist zur Herstellung eines Pflasters zur Verwendung in einem Verfahren zur Erhöhung der Geschwindigkeit des Eindringens in die Haut und / oder des Durchwanderns durch die Haut und / oder der transdermalen Absorptionsrate von einem auf die Haut applizierten pharmazeutischen und / oder kosmetischen Wirkstoff, der eine systemische Wirkung erzielt, wobei die Komponente in einem separaten, gegenüber den wirkstoffhaltigen Konstruktionselementen räumlich getrennten Konstruktionselement vorliegt.

12. Verwendung nach Anspruch 11, **dadurch gekennzeichnet, dass** die Komponente, die zu einer lokalen Temperaturerhöhung und / oder Durchblutungssteigerung der Haut befähigt ist, aus der Gruppe umfassend Rubefacientia, Vasodilatatoren und hyperämisierende Stoffe ausgewählt ist.

13. Verwendung nach Anspruch 11, **dadurch gekennzeichnet, dass** die Komponente, die zu einer lokalen Temperaturerhöhung und / oder Durchblutungssteigerung der Haut befähigt ist, aus der Gruppe umfassend Pelargonsäurevanillylamid, Cayennepfefferölharz, Extract. Fruct. Capsici, Tinct. Capsici, Cayennepfefferextrakt, Cayennepfeffer, Capsaicin, Nicotinsäure, Derivate der Nicotinsäure, Nicotinylalkohol, Nicotinsäurebenzylester, Nicotinamid, Adenosin-Verbindungen, Xanthin, Derivate des Xanthins, Coffein, Amikaextrakt, Arnikablütenextrakt, Pyridyl-3-carbinol und seine Salze, Chamomilla, Phytolacca, Guaiacum, Cinnabaris, Kreosotum, Luffa operculata, Tinct. rhus. toxicodendron, etherische Öle, Menthol, Campher, Nicotinsäureester, Salicylsäureester, Hydroxyethylsalicylat, Methylsalicylat, ortho-Carbamoylphenoxyessigsäure ausgewählt ist.

## Claims

1. Device in the form of a plaster for increasing the rate of penetration into the skin and/or travel through the skin and/or the transdermal absorption rate of an active substance applied to the skin, which plaster is of layered construction, includes at least one layer comprising a component which brings about a local temperature increase and/or circulation increase in the skin, and includes at least one layer which comprises at least one pharmaceutical and/or cosmetic active substance which possesses a systemic action, **characterized in that** said layers are spatially separate from one another.

2. Device according to Claim 1, **characterized in that** it comprises a backing layer that is impermeable to the component which brings about a local temperature increase, or that prevents emergence of this component on the side facing away from the skin.

3. Device according to Claim 1 or 2, **characterized in that** it comprises a pressure sensitive adhesive layer which extends over the full area of the bottom side of the device or is located only at the edge of the bottom side of the device.

4. Device according to any of the preceding claims, **characterized in that** the spatial separation is achieved by means of a horizontal barrier layer, a vertical barrier layer or a gap in material.

5. Device according to any of the preceding claims, **characterized in that** the component which brings about a local temperature increase and/or circulation increase in the skin is selected from the group of rubefacientia and/or vasodilators and/or hyperaemic substances.

6. Device according to any of the preceding claims, **characterized in that** the component which brings about a local temperature increase and/or circulation increase in the skin comprises pelargonic acid vanillyl amide, cayenne pepper oil resin, capsicum fruit extract, tincture of capsicum, cayenne pepper extract, cayenne pepper, capsaicin or a combination thereof.

7. Device according to any of the preceding claims, **characterized in that** the component which brings about a local temperature increase and/or circulation increase in the skin comprises nicotinic acid; derivatives of nicotinic acid, nicotinyl alcohol, benzyl nicotinate, nicotinamide, adenosine compounds; xanthine, derivatives of xanthine, caffeine, arnica extract, arnica blossom extract, pyridyl-3-carbinol and its salts, camomile, phytolacca, guaiacum, cinnabaris, creosotum, Luffa operculata, tincture of Rhus toxicodendron or combinations thereof.

8. Device according to any of the preceding claims, **characterized in that** the component which brings about a local temperature increase and/or circulation increase in the skin comprises essential oils, menthol, camphor, nicotinic esters, salicylic esters, hydroxyethyl salicylate, methyl salicylate, ortho-carbamoylphenoxyacetic acid or combinations thereof.

9. Device according to any of the preceding claims, **characterized in that** the systemic active substance applied to the skin is a pharmaceutical active substance selected from the group consisting of opiates, morphine, diamorphine, buprenorphine, fentanyl, oestrogens, 17β-estradiol, hormones, testosterone, norethisterone, gestagens, peptides, insulin, analgesics and alkaloids.

10. Device according to any of the preceding claims, **characterized in that** around an inner segment accommodating the at least one pharmaceutical and/or cosmetic active substance an outer segment is disposed which accommodates the component which brings about a local temperature increase and/or circulation increase in the skin.

11. Use of a component capable of local temperature increase and/or circulation increase in the skin for producing a plaster for use in a method of increasing the rate of penetration into the skin and/or of travel through the skin and/or the transdermal absorption rate of a pharmaceutical and/or cosmetic active substance applied to the skin and producing a systemic effect, the component being present in a discrete structural element spatially separate from the structural elements comprising active substance.

12. The use according to Claim 11, **characterized in that** the component capable of local temperature increase and/or circulation increase in the skin is selected from the group comprising rubefacientia, vasodilators and hyperaemic substances.

13. Use according to Claim 11 **characterized in that** the component capable of local temperature increase and/or circulation increase in the skin is selected from the group consisting of pelargonic acid vanillyl amide, cayenne pepper oil resin, capsicum fruit extract, tincture of capsicum, cayenne pepper extract, cayenne pepper, capsaicin, nicotinic acid, derivatives of nicotinic acid, nicotinyl alcohol, benzyl nicotinate, nicotinamide, adenosine compounds, xanthine, derivatives of xanthine, caffeine, arnica extract, amica blossom extract, pyridyl-3-carbinol and its salts, camomile, phytolacca, guaiacum, cinnabaris, creosotum, Luffa operculata, tincture of Rhus toxicodendron, essential oils, menthol, camphor, nicotinic esters, salicylic esters, hydroxyethyl salicylate, methyl salicylate, ortho-carbamoylphenoxyacetic acid.

## Revendications

1. Dispositif sous forme d'un pansement adhésif pour augmenter la vitesse de pénétration dans la peau et/ou le passage à travers la peau et/ou le taux d'absorption transdermique d'une substance active appliquée sur la peau, dans lequel le pansement a une structure en couches, comprend au moins une couche contenant un constituant qui entraîne une élévation locale de la température et/ou une augmentation de l'irrigation sanguine de la peau et comprend au moins une couche contenant une substance active pharmaceutique et/ou cosmétique possédant une activité systémique, **caractérisé en ce que** lesdites couches sont séparées l'une de l'autre dans l'espace.

2. Dispositif selon la revendication 1, **caractérisé en ce qu'**il contient une couche postérieure qui est imperméable au constituant entraînant une augmentation locale de la température ou qui empêche la sortie de ce constituant vers le côté opposé à la peau.

3. Dispositif selon la revendication 1 ou 2, **caractérisé en ce qu'**il contient une couche adhésive de fixation qui s'étend sur toute la surface de la face inférieure du dispositif ou qui ne se trouve que sur le bord de la face inférieure du dispositif.

4. Dispositif selon l'une des revendications précédentes, **caractérisé en ce que** la séparation spatiale est obtenue par une couche isolante horizontale, une couche isolante verticale ou un vide de matière.

5. Dispositif selon l'une des revendications précédentes, **caractérisé en ce que** le constituant qui entraîne une élévation locale de la température et/ou une augmentation de l'irrigation sanguine de la peau est choisi dans le groupe des rubéfiants et/ou des vasodilatateurs et/ou des substances hyperémiantes.

6. Dispositif selon l'une des revendications précédentes, **caractérisé en ce que** le constituant qui entraîne une élévation locale de la température et/ou une augmentation de l'irrigation sanguine de la peau contient du vanillylamide de l'acide pélargonique, de l'oléorésine de poivre de Cayenne, un extrait de piment fort, de la teinture de piment fort, un extrait de poivre de Cayenne, du poivre de Cayenne, de la capsaïcine ou une de leurs combinaisons.

7. Dispositif selon l'une des revendications précédentes, **caractérisé en ce que** le constituant qui entraîne une élévation locale de la température et/ou une augmentation de l'irrigation sanguine de la peau contient de l'acide nicotinique, des dérivés de l'acide nicotinique, de l'alcool nicotinique, du nicotinate de benzyle, du nicotinamide, des composés d'adénosine, de la xanthine, des dérivés de xanthine, de la caféine, de l'extrait d'arnica, de l'extrait de fleurs d'arnica, du pyridyl-3-carbinol et ses sels, de la camomille, de la phytolaque, du gaiac, du cinabre, de la créosote, de la Luffa operculata, de la teinture de Rhus toxicodendron ou leurs combinaisons.

8. Dispositif selon l'une des revendications précédentes, **caractérisé en ce que** le constituant qui entraîne une élévation locale de la température et/ou une augmentation de l'irrigation sanguine de la peau contient des huiles éthérées, du menthol, du camphre, des esters de l'acide nicotinique, des esters de l'acide salicylique, du salicylate d'hydroxyéthyle, du salicylate de méthyle, de l'acide ortho-carbamoylphénoxyacétique ou leurs combinaisons.

9. Dispositif selon l'une des revendications précédentes, **caractérisé en ce que** la substance active systémique appliquée sur la peau est une substance active pharmaceutique du groupe comprenant des opiacés, la morphine, la diamorphine, la buprénorphine, le fentanyl, des oestrogènes, le 17β-estradiol, des hormones, la testostérone, la noréthistérone, des gestagènes, des peptides, l'insuline, des analgésiques et des alcaloïdes.

10. Dispositif selon l'une des revendications précédentes, **caractérisé en ce qu'**un segment extérieur, contenant le constituant qui entraîne une élévation. locale de la température et/ou une augmentation de l'irrigation sanguine de la peau, est disposé autour d'un segment interne qui contient la ou les substances actives pharmaceutiques et/ou cosmétiques.

11. Utilisation d'un constituant capable de faire monter la température locale et/ou d'augmenter l'irrigation sanguine de la peau pour la préparation d'une pansement adhésif à utiliser dans un procédé pour augmenter la vitesse de pénétration dans la peau et/ou le passage à travers la peau et/ou le taux d'absorption transdermique d'une substance active pharmaceutique et/ou cosmétique appliquée sur la peau et ayant une activité systémique, le constituant se trouvant dans un élément structurel individuel séparé dans l'espace de l'élément structurel contenant la substance active.

12. Utilisation selon la revendication 11, **caractérisée en ce que** le constituant capable de faire monter la température locale et/ou d'augmenter l'irrigation sanguine de la peau est choisi dans le groupe comprenant les rubéfiants, les vasodilatateurs et les substances hyperémiantes.

13. Utilisation selon la revendication 11, **caractérisée en ce que** le constituant capable de faire monter la température locale et/ou d'augmenter l'irrigation sanguine de la peau est choisi dans le groupe comprenant du vanillylamide de l'acide pélargonique, de l'oléorésine de poivre de Cayenne, un extrait de piment fort, de la teinture de piment fort, un extrait de poivre de Cayenne, du poivre de Cayenne, de la capsaïcine, de l'acide nicotinique, des dérivés de l'acide nicotinique, de l'alcool nicotinique, du nicotinate de benzyle, du nicotinamide, des composés d'adénosine, de la xanthine, des dérivés de xanthine, de la caféine, de l'extrait d'arnica, de l'extrait de fleurs d'arnica, du pyridyl-3-carbinol et ses sels, de la camomille, de la phytolaque, du gaiac, du cinabre, de la créosote, de la Luffa operculata, de la teinture de Rhus toxicodendron, des huiles éthérées, du menthol, du camphre, des esters de l'acide nicotinique, des esters de l'acide salicylique, du salicylate d'hydroxyéthyle, du salicylate de méthyle, de l'acide ortho-carbamoylphénoxyacétique.
